# EUROPEAN PATENT SPECIFICATION

(11) **EP 4 398 872 B1**
(45) Date of publication and mention of the grant of the patent: **22.10.2025**
(21) Application number: 22765583.4
(22) Date of filing: 08.09.2022
(51) Int. Cl.: A61K 9/00, A61K 9/08, A61K 9/19, A61K 38/26, A61K 47/18, A61K 47/26

(54) **PROCESS FOR FORMULATING COMPOSITIONS COMPRISING GLUCAGON-LIKE-PEPTIDE-2 (GLP-2) ANALOGUES**
VERFAHREN ZUR FORMULIERUNG VON ZUSAMMENSETZUNGEN MIT GLUCAGON-LIKE-PEPTID-2(GLP-2)-ANALOGA
PROCÉDÉ DE FORMULATION DE COMPOSITIONS COMPRENANT DES ANALOGUES DU PEPTIDE-2 DE TYPE GLUCAGON (GLP-2)

(30) Priority: 10.09.2021 EP 21196169
(43) Date of publication of application: 17.07.2024
(73) Proprietor: Zealand Pharma A/S, 2860 Søborg (DK)
(72) Inventor: MØLLER, Eva Horn, 2860 Søborg (DK); LUNDQVIST, Joakim, 2860 Søborg (DK)
(74) Representative: Mewburn Ellis LLP
(86) International application number: PCT/EP2022/074961
(87) International publication number: WO 2023/036862

(56) References cited:
- EP-A1- 3 628 682
- EP-A1- 3 628 683
- KAROLINA L. ZAPADKA ET AL: "Factors affecting the physical stability (aggregation) of peptide therapeutics", INTERFACE FOCUS, vol. 7, no. 6, 20 October 2017 (2017-10-20), GB, pages 20170030, XP055598856, ISSN: 2042-8898, DOI: 10.1098/rsfs.2017.0030

## Description

### Field of the Invention

The present invention relates to processes for manufacturing stable liquid pharmaceutical formulations comprising a glucagon-like peptide 2 (GLP-2) analogue, and in particular to processes for producing formulations that comprise ZP1848 (glepaglutide) and/or ZP1846 (elsiglutide), including their metabolites.

### Background of the Invention

Human GLP-2 is a 33-amino-acid peptide with the following sequence: Hy-His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp-OH (SEQ ID NO: 10). It is derived from specific post-translational processing of proglucagon in the enteroendocrine L cells of the intestine and in specific regions of the brainstem. GLP-2 binds to a single G-protein-coupled receptor belonging to the class II glucagon secretin family.

GLP-2 has been reported to induce significant growth of the small intestinal mucosal epithelium via the stimulation of stem cell proliferation in the crypts, and by inhibition of apoptosis in the villi (Drucker et al., 1996, Proc. Natl. Acad. Sci. USA 93: 7911-7916). GLP-2 also has growth effects on the colon. Furthermore, GLP-2 inhibits gastric emptying and gastric acid secretion (Wojdemann et al., 1999, J. Clin. Endocrinol. Metab. 84: 2513-2517), enhances intestinal barrier function (Benjamin et al., 2000, Gut 47: 112-119), stimulates intestinal hexose transport via the upregulation of glucose transporters (Cheeseman, 1997, Am. J. Physiol. R1965-71), and increases intestinal blood flow (Guan et al., 2003, Gastroenterology, 125: 136-147).

It has been recognised in the art that glucagon-like peptide-2 receptor analogues have therapeutic potential for the treatment of intestinal diseases. However, the native hGLP-2, a 33 amino acid gastrointestinal peptide, is not a useful in a clinical setting due to its very short half-life in humans of around 7 minutes for full length GLP-2 [1-33] and 27 minutes for truncated GLP-2 [3-33]. In large part, the short half-life is due to degradation by the enzyme dipeptidylpeptidase IV (DPP-IV). Accordingly, there have been attempts in the art to develop GLP-2 receptor agonists with better pharmacokinetic characteristics, in particular to improve the half-life of GLP-2 molecules. By way of example, GLP-2 analogues with substitutions have been suggested such as e.g. GLP-2 analogues containing Gly substitution at position 2 ([hGly2] GLP-2, teduglutide) which increases the half-life from seven minutes (native GLP-2) to about two hours. Acylation of peptide drugs with fatty acid chains has also proven beneficial for prolonging systemic circulation as well as increasing enzymatic stability without disrupting biological potency. However, while these attempts have improved the pharmacokinetics of GLP-2 analogues, and they are sometimes described in the art as "long acting", it must be kept in mind that this is in comparison to native hGLP-2 with half-lives of the order of several hours, rather than minutes. This in turn means that the GLP-2 analogues still need to be administered to patients one or more times per day. Teduglutide is approved for treatment of short bowel syndrome under the names Gattex (in the US) and Revestive (in Europe).

WO 2006/117565 (Zealand Pharma A/S) describes GLP-2 analogues which comprise one or more substitutions as compared to [hGly2]GLP-2 and which improved biological activity *in vivo* and/or improved chemical stability, e.g. as assessed in *in vitro* stability assays. Among the molecules disclosed in WO 2006/117565 are ZP1848 (glepaglutide) and ZP1846 (elsiglutide), along with their medical uses for the treatment of stomach and bowel-related disorders and for ameliorating side effects of chemotherapy and radiation therapy. Dosage regimes for GLP-2 analogues including ZP1848 and ZP1846 and their metabolites are described in WO 2018/229252. Ready to use formulations of ZP1848 and ZP1846 are described in WO 2020/065064 and WO 2020/065063.

Zapadka et al. (Interface Focus 7: 20170030. http://dx.doi.org/10.1098/rsfs.2017.0030) describes factors affecting the physical stability of peptide therapeutics, in particular their tendency to aggregate.

It remains a problem in this area to improve the manufacturing processes used for the formulation of GLP-2 analogues after synthesis, in particular to address problems that arise from the unusual properties of these molecules. It would also be a goal in the area of GLP-2 analogue formulation to provide improved processes for making formulations of GLP-2 analogues formulations, in particular for use in delivery devices such as pre-filled syringes, infusion pumps, wearable injectors or auto-injectors.

### Summary of the Invention

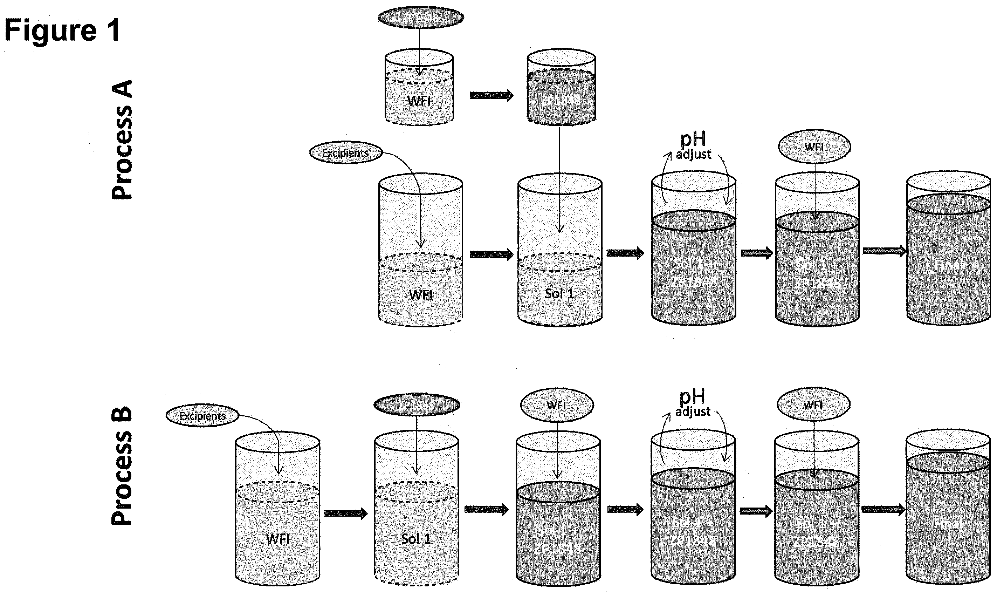

Broadly, the present invention relates to processes for manufacturing pharmaceutical formulations of GLP-2 analogues, and in particular processes for manufacturing stable liquid pharmaceutical formulations comprising ZP1848 (glepaglutide) and ZP1846 (elsiglutide) and/or their metabolites. Accordingly, the present invention concerns the development of a new process ("Process B" or "one-pot formulation process") that ameliorates technical issues from the previous manufacturing process, "Process A" or "two-pot formulation process" or "two-solution formulation process", see Figure 1. Until now, ZP1848 and ZP1846 drug substances have been manufactured using the "Process A". However, Process A using ZP1848 or ZP1846 leads to some technical challenges associated with excessive foaming when the GLP-2 analogue is dissolved when added to other components of the formulation, the formation of hard lumps of drug substance during dissolution, deviations from visual controls and the formation of higher peptide oligomers. Foaming and lump formation and the need to use manual stirring can be problematic in the scale up of the manufacturing process because lumps of the active pharmaceutical ingredient (API) can be hidden in the foam layer, causing failure of the important visual process control for clear solution and dissolved drug substance.

Process A is a traditional formulation process for peptides. During formulation development, it is common in the art to use a stock dissolution of the peptide in water as this enables the handling of the peptide that way in a safe and easy manner and because it is easier to vary combinations of different excipients. Process A works well in small scale. Also, for peptides in general, where the pl is crossed during formulation, Process A is preferred to avoid local pl and precipitations of the peptide that can be hard or impossible to redissolve in the formulation process.

Without being bound by any specific explanation, the present inventors believe that the problematic and unusual behaviour of ZP1848 and ZP1846 leading to the observed foaming and lump formation when using Process A is linked to the specific physicochemical properties of ZP1848 and ZP1846, especially the amphiphilic character of the peptide structure. This property of ZP1848 and ZP1846 leads to an unusual propensity to form gels at higher concentrations, e.g. in solid/water interfaces during dissolution, which may in turn contribute to the unwanted lump formation during the formulation process. In Process A for ZP1848 and related GLP-2 analogues, these issues combine to lead to a particularly slow and difficult dissolution of the drug substance, in particular at higher concentrations of the GLP-2 analogue in the final formulation (such as 20 mg/mL or higher), as these require dissolution of the drug substance as a 50 mg/mL solution (i.e. at about 40% of the final volume of the formulation). In this dissolution process, extreme foaming occurs and lumps are formed. Lumps of the API will then be hidden in the foam layer, causing failure of the important visual process control for clear solution and dissolved drug substance.

Thus, Process A necessitates a relatively high peptide concentration in the API solution. At high peptide concentrations (e.g. at least 20 mg/mL or preferably at least 50 mg/mL or higher), ZP1848 and ZP1846 can be sensitive to vigorous stirring, e.g. by mechanical means as typically used in peptide formulation processes, and hence manual stirring of the peptide drug substance during the dissolution step has been used in Process A. Manual stirring is not compatible with upscaling to industrial scale, and further it is a subjective process that cannot be validated in a process validation.

In Process A, the excipients and the active ingredient are dissolved in separate solutions and tanks in parallel processes, with the two solutions of the excipients and the peptide drug substance, respectively, subsequently being mixed, prior to the optional steps where the volume and/or the pH of the formulation (i.e. the mixed solution) are adjusted to provide the final formulation ready for packaging and clinical uses.

The options for circumventing the technical issues of Process A as described above are further limited due to the concentration of mannitol. The excipient solution contains mannitol at concentrations close to its upper solubility limit, and therefore it is not possible to solve the issues of Process A by reducing the volume of the excipient solution and concomitantly increasing the volume available for the API solution.

In summary, this means that the current Process A has technical issues, and that scaling up from the current 10 litre batch size is unfeasible using Process A.

In the present invention, a "one-pot" formulation process or "Process B" means that only one tank or vessel is employed, in which water, excipients and the drug substance are added in sequence in any particular order, including optional steps of adjustment of the volume and/or the pH of the formulation to provide the final formulation ready for packaging and clinical uses. This may be contrasted, as described above, with a "two-pot formulation" process in which the parallel dissolution of API and excipients in two solutions becomes a limit. Due to the proportions of water, the excipients and drug substance in Process B, only one pot/tank can be used, which makes this process simpler, easier to handle, more robust to deviations, practicable to upscale, and results in a final formulation with improved stability.

In Process B described in the examples below excipients (mannitol, histidine) are dissolved in for example 80% of the final volume; GLP-2 analogue drug substance is added as a dry powder to give for example approx. 25 mg/ml; the volume is adjusted to 90%; pH is adjusted as necessary; and the volume is adjusted to 100% to give the final glepaglutide (20 mg/mL) formulation.

Accordingly, in a first aspect, the present invention provides a process for manufacturing a stable liquid pharmaceutical formulation comprising a glucagon-like peptide 2 (GLP-2) analogue, wherein the GLP-2 analogue is represented by the formula:
ZP1848 H-HGEGTFSSELATILDALAARDFIAWLIATKITDKKKKKK-NH₂ (SEQ ID NO: 1) or
ZP1846 H-HGEGSFSSELSTILDALAARDFIAWLIATKITDKKKKKK-NH₂(SEQ ID NO: 7);
or a pharmaceutically acceptable salt thereof,
wherein the liquid pharmaceutical formulation comprises the GLP-2 analogue at a concentration of about 2 mg/mL to about 30 mg/mL and excipients comprising (i) a histidine buffer present at a concentration of about 5 mM to about 50 mM, (ii) mannitol as a non-ionic tonicity modifier present at a concentration of about 90 mM to about 360 mM and with (iii) arginine q.s. to provide a formulation having a pH of about 6.6 to about 7.4;
wherein the process comprises:
   (a) mixing the excipients with a first volume of water for injection to provide an excipient solution at 70-90% of the volume of a final liquid formulation;
   (b) adding the GLP-2 analogue drug substance to the excipient solution to provide a solution of the excipients and the GLP-2 analogue at 70-90% of the volume of a final liquid formulation; and
   (c) adjusting the pH and the volume of the liquid composition as needed to provide a liquid formulation at 100% of the volume of the final liquid pharmaceutical formulation;
wherein the process provides a final aqueous liquid pharmaceutical formulation comprising a glucagon-like peptide 2 (GLP-2) analogue.

In some aspects, the process does not require stirring when the GLP-2 solution composition is added to the excipient solution, helping to minimise the adverse effects of excessive foaming.

In general, in step (b) the GLP-2 analogue drug substance is added as a lyophilized composition, typically added in 1-5 portions, preferably in 3 portions. Typically the GLP-2 analogue drug substance will be in the form of a powder. Alternatively or additionally, adjusting the volume of the liquid composition in step (c) comprises a first addition of water for injection to adjust the pH and a second addition of water for injection to increase the volume of the liquid composition towards the final volume, optionally wherein adjusting the pH of the liquid composition is carried out by adding arginine and/or acetic acid.

Advantageously, in the manufacturing process, the excipient solution is at about 80% the volume of a final liquid formulation and the first and second additions each add about 10% of the volume of a final liquid formulation. In alternative cases, the excipient solution is at about 75% the volume of a final liquid formulation and the first and second additions each add about 15% and about 10% of the volume of a final liquid formulation.

In some cases, the manufacturing process further comprises sterile filtering the final liquid pharmaceutical formulation (e.g. to remove microorganism) and/or dividing the final liquid pharmaceutical formulation into doses and/or quality control testing the final liquid pharmaceutical formulation and comparing results of the quality control testing with a reference, for example a reference which is the same or consistent with regulatory approval or literature.

The manufacturing processes of the present invention also enable the scale up of the previously used pilot scale production of the GLP-2 analogue liquid formulations. This enables batch size of more than 10 litres of the final liquid pharmaceutical formulation to be produced, for example batches of 15 litres, 20 litres, 30 litres, 40 litres or even 50 litres of the final liquid pharmaceutical formulation, for example in volume ranges of between 10-50 litres of final liquid pharmaceutical formulation, and optionally a batch size of 10-20 litres of final liquid pharmaceutical formulation, and optionally a batch size of 20-50 litres of final liquid pharmaceutical formulation. In some cases, the process is carried out in a 10 litre or a 20 litre tank.

In some cases, mechanical stirring, especially gentle stirring, may be used in step (b) to dissolve the GLP-2 analogue, e.g. using a stirrer wing.

In some cases, one or more of the step in the process may be carried out under nitrogen.

The formation of oligomers and/or impurities in glepaglutide drug product is reduced in batches prepared with nitrogen gas compared to batches with oxygen or no gas. By way of example, this may be carried out by purging the formulation with nitrogen or by carrying out the process under nitrogen gas overlay.

As the formations manufactured using the processes of the present invention are for administration to patients (especially human patients), the processes may comprise performing a visual control after step (a) to determine whether the excipients have (fully) dissolved and/or performing a visual control after step (b) to determine whether the GLP-2 analogue and the excipients have substantially completely dissolved and/or performing a visual control after step (c) to determine whether the process has produced a solution of the GLP-2 analogue and excipients.

In some cases, step (b) comprises washing a vessel or a transfer bag or container that contained the lyophilized GLP-2 analogue to remove any residual GLP-2 analogue. Alternatively or additionally, the addition of the GLP-2 analogue in step (b) reduces foaming of the composition comprising the GLP-2 analogue and the excipients and/or formation of residual lumps of the GLP-2 analogue, e.g. to secure visual control of clear solution at the end of the process, and/or the addition of the GLP-2 analogue in step (b) reduces the level of covalently linked high molecular weight species in the final liquid pharmaceutical formulation.

In common with the aim of providing storage stable formulations of the GLP-2 analogues, the processes of the present invention lead to formulations that are stable for at least 18 months when stored at 2-8°C. By way of illustration, the formulations may comprise the GLP-2 analogue, or the pharmaceutically acceptable salt thereof; at a concentration of about 20 mg/mL, histidine buffer at a concentration of about 15 mM, mannitol at a concentration of about 230 mM, and arginine q.s. to provide a pH of about 7.0. Generally, the histidine buffer is L-histidine and the mannitol is D-mannitol.

The processes of the present invention may also entail downstream steps, for example loading the formulation into a pre-filled syringe, an injection pen or an injector device.

In some case, the processes of the present invention reduce the formation of covalently bonded oligomeric products of a glucagon-like peptide 2 (GLP-2) analogue in the stable liquid pharmaceutical formulation comprising a GLP-2 analogue.

In a further aspect, the present invention provides the use of a formulation for reducing the formation of covalently bonded oligomeric products of a glucagon-like peptide 2 (GLP-2) analogue, wherein the GLP-2 analogue is represented by the formula:
ZP1848 H-HGEGTFSSELATILDALAARDFIAWLIATKITDKKKKKK-NH₂ (SEQ ID NO: 1) or
ZP1846 H-HGEGSFSSELSTILDALAARDFIAWLIATKITDKKKKKK-NH₂(SEQ ID NO: 7); or a pharmaceutically acceptable salt thereof;
wherein the liquid pharmaceutical formulation comprises the GLP-2 analogue at a concentration of about 2 mg/mL to about 30 mg/mL and excipients comprising (i) a histidine buffer present at a concentration of about 5 mM to about 50 mM, (ii) mannitol as a non-ionic tonicity modifier present at a concentration of about 90 mM to about 360 mM and with (iii) arginine q.s. to provide a formulation having a pH of about 6.6 to about 7.4;
wherein the use comprises:
   (a) mixing the excipients with a first volume of water for injection to provide an excipient solution at 70-90% of the volume of a final liquid formulation;
   (b) adding the GLP-2 analogue drug substance to the excipient solution to provide a solution of the excipients and the GLP-2 analogue at 70-90% of the volume of a final liquid formulation; and
   (c) adjusting the pH and the volume of the liquid composition as needed to provide a liquid formulation at 100% of the volume of the final liquid pharmaceutical formulation;
wherein the process provides a final aqueous liquid pharmaceutical formulation comprising a glucagon-like peptide 2 (GLP-2) analogue.

In some embodiments, the formulation contains 5% or less of the GLP-2 analogue in the form of covalently bonded oligomeric products. Alternatively or additionally, the total acetate concentration arising from the GLP-2 analogue in the formulation is less than or equal to 11% acetate per mg GLP-2 analogue. Alternatively or additionally, formation of covalently linked oligomers of the GLP-2 analogue is inversely dependent on the concentration of the GLP-2 analogue in the formulation.

Embodiments of the present invention will now be described by way of example and not limitation with reference to the accompanying figures. However, various further aspects and embodiments of the present invention will be apparent to those skilled in the art in view of the present disclosure.

While the present invention has been described in conjunction with the embodiments described above, many equivalent modifications and variations will be apparent to those skilled in the art when given this disclosure. Accordingly, the embodiments of the invention set forth are considered to be illustrative and not limiting.
"and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Brief Description of the Figures

**Figure 1** shows a flowchart of the new formulation "one-pot" Process B compared to the traditional "two-pot" Process A.

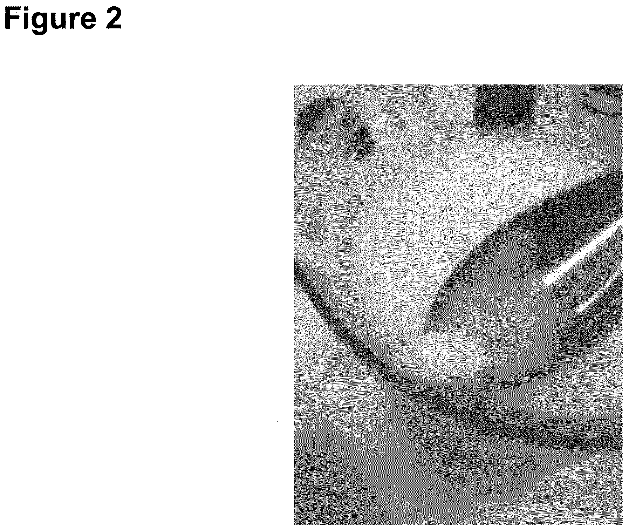

**Figure 2** show an image of foaming that results during mixing in a two-pot Process A.

### Detailed Description of the Invention

Unless specified otherwise, the following definitions are provided for specific terms, which are used in the above written description.

Throughout the description and claims the conventional one-letter and three-letter codes for natural amino acids are used. All amino acid residues in peptides of the invention are preferably of the L-configuration, However, D-configuration amino acids may also be present.

### GLP-2 Analogues

Also described are glucagon-like peptide 2 (GLP-2) analogues that can be formulated using the methods of the present invention are represented by the formula:

R¹-Z¹-His-Gly-Glu-Gly-X5-Phe-Ser-Ser-Glu-Leu-X11-Thr-Ile-Leu-Asp-Ala-Leu-Ala-Ala-Arg-Asp-Phe-Ile-Ala-Trp-Leu-Ile-Ala-Thr-Lys-Ile-Thr-Asp-Z²-R²

wherein:
R¹ is hydrogen, C₁₋₄ alkyl (e.g. methyl), acetyl, formyl, benzoyl or trifluoroacetyl;
X5 is Ser or Thr;
X11 is Ala or Ser;
R² is NH₂ or OH; and
Z¹ and Z² are independently absent or a peptide sequence of 1-6 amino acid units of Lys;
or a pharmaceutically acceptable salt thereof.

Z¹ and Z² are independently present and/or absent or a peptide sequence of 1-6 amino acid units of Lys, i.e. 1, 2, 3, 4, 5 or 6 Lys residues. The Lys residues may have either D- or L-configuration, but preferably have an L-configuration. Particularly preferred sequences Z are sequences of four, five or six consecutive lysine residues, and particularly six consecutive lysine residues. Exemplary sequences Z are shown in WO 01/04156.

Also described are GLP-2 analogues which in the above formulae, X5 is Thr and/or X11 is Ala. Examples of these glucagon-like peptide 2 (GLP-2) analogues include:
ZP1848 H-HGEGTFSSELATILDALAARDFIAWLIATKITDKKKKKK-NH₂ (SEQ ID NO: 1)

In some embodiments of the present invention, the glucagon-like peptide 2 (GLP-2) analogue is:
ZP1848 H-HGEGTFSSELATILDALAARDFIAWLIATKITDKKKKKK-NH₂ (SEQ ID NO: 1).

That is, the GLP-2 analogue is represented by the formula:

H-His¹-Gly²-Glu³ -Gly⁴-Thr⁵-Phe⁶-Ser⁷-Ser⁸-Glu⁹-Leu¹⁰-Ala¹¹-Thr¹²-Ile¹³-Leu¹⁴-Asp¹⁵-Ala¹⁶-Leu¹⁷-Ala¹⁸-Ala¹⁹-Arg²⁰-Asp²¹-Phe²²-Ile²³-Ala²⁴-Trp²⁵-Leu²⁶-Ile²⁷-Ala²⁸-Thr²⁹-Lys³⁰-Ile³¹-Thr³²-Asp³³-Lys³⁴-Lys³⁵-Lys³⁶-Lys³⁷-Lys³⁸-Lys³⁹-NH₂.

It will be understood that the N-terminal "H-" indicates a free N-terminal amine (NH₂-) group. The C-terminal "NH₂-" indicates a C-terminal amide group. The terms ZP1848 and glepaglutide may be used interchangeably.

Also described are GLP-2 analogues which in the above formula X5 is Ser and/or X11 is Ser. Examples of these glucagon-like peptide 2 (GLP-2) analogues include:
ZP1846 H-HGEGSFSSELSTILDALAARDFIAWLIATKITDKKKKKK-NH₂(SEQ ID NO: 7);

In an embodiment of the present invention, the glucagon-like peptide 2 (GLP-2) analogue is ZP1846 H-HGEGSFSSELSTILDALAARDFIAWLIATKITDKKKKKK-NH₂ (SEQ ID NO: 7).

That is, the GLP-2 analogue is represented by the formula:

H-His¹-Gly²-Glu³-Gly⁴-Ser⁵-Phe⁶-Ser⁷-Ser⁸-Glu⁹-Leu¹⁰-Ser¹¹-Thr¹²-Ile¹³-Leu¹⁴-Asp¹⁵-Ala¹⁶-Leu¹⁷-Ala¹⁸-Ala¹⁹-Arg²⁰-Asp²¹-Phe²²-Ile²³-Ala²⁴-Trp²⁵-Leu²⁶-Ile²⁷-Ala²⁸-Thr²⁹-Lys³⁰-Ile³¹-Thr³²-Asp³³-Lys³⁴-Lys³⁵-Lys³⁶-Lys³⁷-Lys³⁸-Lys³⁹-NH₂.

It will be understood that the N-terminal "H-" indicates a free N-terminal amine (NH₂-) group. The C-terminal "NH₂-" indicates a C-terminal amide group. The terms ZP1846 and elsiglutide may be used interchangeably.

The present invention is directed to methods of making and purifying GLP2 analogues. These peptides are intended for use as drug substances. The invention includes GLP analogues obtained by the methods of the invention.

It should be understood that the peptides (drug substance) of the invention might be provided in the form of a salt. Accordingly, it will be understood that, after purification and optional further steps, the peptide may be finally obtained as a salt. Salts include pharmaceutically acceptable salts, such as acid addition salts and basic salts. Examples of acid addition salts include hydrochloride salts, citrate salts, chloride salts and acetate salts. Preferably, the salt is acetate. Examples of basic salts include salts where the cation is selected from alkali metals, such as sodium and potassium, alkaline earth metals, such as calcium, and ammonium ions ⁺N (R³) ₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences",17th edition. Ed. Alfonso R. Gennaro (Ed.), Mark Publishing Company, Easton, PA, U.S.A., 1985 and more recent editions, and in the Encyclopaedia of Pharmaceutical Technology.

In preferred embodiments, the acetate salt of a GLP-2 analogue of the invention is selected from the group consisting of ZP1848-acetate, ZP2711-acetate, ZP2469-acetate, ZP1846-acetate and ZP2242-acetate. In the present context, the term "ZP1848-acetate" refers to the ZP1848 molecule is in the form of an acetate salt. The acetate salts of GLP-2 analogues may be represented by the formula (GLP-2 analogue), x(CH₃COOH) where x is 1.0 to 8.0, i.e. where x is 1.0, 2.0, 3.0, 4.0, 5.0, 6.0, 7.0 or 8.0. In any composition of the acetate salts of the GLP-2 analogues, there may be molecules with different number of acetate molecules so that x is not necessarily a whole integer. In some cases, x is from 4.0 to 8.0, x is from 6.0 to 8.0, or x is from 4.0 to 6.5. In some cases, x is from 4.0 to 6.0, x is from 2.0 to 6.0, x is from 2.0 to 7.0, x is from 3.0 to 6.0, x is from 4.0 to 6.0, or x is 4.0 to 8.0. Further discussion of acetate salts of GLP-2 analogues as defined in the invention may be found in WO 2020/265064.

In a preferred embodiment, the GLP-2 analogue is finally obtained as ZP1848-acetate or H-HGEGTFSSELATILDALAARDFIAWLIATKITDKKKKKK-NH₂ acetate (SEQ ID NO: 1) or (H-HGEGTFSSELATILDALAARDFIAWLIATKITDKKKKKK-NH₂), x(CH₃COOH) where x is 1.0 to 8.0.

A solid composition comprising an acetate salt of a glucagon-like peptide 2 (GLP-2) analogue may be obtained, for example by lyophilization. The solid compositions are useful for formulating with the excipients used to make liquid formulations. For example, a solid composition comprising an acetate salt of a glucagon-like peptide 2 (GLP-2) analogue having the formula:
(H-HGEGTFSSELATILDALAARDFIAWLIATKITDKKKKKK-NH₂), x(CH₃COOH) where x is 1.0 to 8.0
may be obtained.

An upper limit of 8.0 acetate molecules per GLP-2 analogue equates to an acetate content of less than 11% acetate and may be formulated to have a viscosity between 0.8 and 2.0 mPa/sec measured at 25°C.

The range of the number of acetate molecules associated with each molecule of the GLP-2 analogues defines a molecular weight range for this component of the formulation. For example, for the acetate salts of ZP1848, the range of the number of acetate molecules associated with each molecule of the GLP-2 analogues defines a molecular weight range of the ZP1848-acetate. By way of example, 1 acetate equivalent with each molecule of ZP1848 provides a molecular weight = 4316 + 60 = 4376 Da. Accordingly, the molecular weights for increasing acetate equivalents with ZP1848 are as follows: 1 acetate equivalent = 4376 Da; 2 acetate equivalents = 4436 Da; 3 acetate equivalents = 4496 Da; 4 acetate equivalents =4556 Da; 5 acetate equivalents = 4616 Da; 6 acetate equivalents = 4676 Da; 7 acetate equivalents = 4736 Da and 8 acetate equivalents = 4796 Da. This in turn defines molecular weight ranges as follows: 1-8 acetate equivalents = 4376 Da - 4796 Da; 4-8 acetate equivalents = 4556 Da - 4796 Da and 6-8 acetate equivalents = 4676 Da - 4796 Da. Further discussion of acetate salts of GLP-2 analogues as defined in the invention may be found in WO 2020/265064.

### Formulations of the GLP-2 analogues

The formulations of the GLP-2 analogues that may be made using the processes of the present invention are the ready-to-use formulation disclosed in WO 2020/065064 using Process A. The term "ready-to-use" as used herein refers to a formulation that does not require constitution or dilution with a prescribed amount of diluent, e.g., water for injection or other suitable diluent, before use by the designated route of administration.

These liquid formulations of the GLP-2 analogues include a buffer, a non-ionic tonicity modifier and arginine q.s. to provide the pH of the final formulation. In accordance with normal pharmaceutical practice, the formulations are sterile and/or free from reducing agent. In some cases, the liquid formulations are aqueous, liquid formulations.

A preferred stable liquid formulation disclosed in WO 2002/065064 wherein the liquid pharmaceutical formulation comprises the GLP-2 analogue at a concentration of about 2 mg/mL to about 30 mg/mL and excipients comprising (i) a histidine buffer present at a concentration of about 5 mM to about 50 mM, (ii) mannitol as a non-ionic tonicity modifier present at a concentration of about 90 mM to about 360 mM and with (iii) arginine q.s. to provide a formulation having a pH of about 6.6 to about 7.4.

The term "buffer" as used herein denotes a pharmaceutically acceptable excipient which stabilizes the pH of a pharmaceutical formulation. In the formulations produced by the present invention the buffer is a histidine buffer, e.g. L-histidine. Generally, the histidine buffer will be present at a concentration of about 5 mM to about 50 mM, more preferably at a concentration of about 5 mM to about 25 mM, and most preferably at a concentration of about 15 mM.

The term "tonicity modifier" as used herein denotes pharmaceutically acceptable tonicity agents that are used to modulate the tonicity of the formulation. The formulations produced by the present invention are preferably isosmotic, that is they have an osmotic pressure that is substantially the same as human blood serum. The tonicity modifiers used in the formulations produced according to the present invention is mannitol, e.g. D-mannitol. The concentration of the tonicity modifier will be dependent on the concentration of other components of the formulation, especially where the formulation is intended to be isosmotic. Typically, the non-ionic tonicity modifier will be employed at a concentration of about 90 mM to about 360 mM, more preferably at a concentration of about 150 mM to about 250 mM, and most preferably at a concentration of about 230 mM.

Generally, the components and amounts of the liquid formulations produced by the present invention are chosen to provide a formulation with a pH of about 6.6 to about 7.4, more preferably a pH of about 6.8 to about 7.2, and most preferably a pH of about 7.0. Arginine may be added *quantum sufficit* (*q.s.*) to adjust pH so that it is within a desired pH range. It is preferred that the pH adjustment is not done using hydrochloric acid or sodium hydroxide.

In a preferred embodiment, the liquid formulations produced according to processes of the present invention comprises ZP1848-acetate or H-HGEGTFSSELATILDALAARDFIAWLIATKITDKKKKKK-NH₂ acetate (SEQ ID NO: 1) or ZP1846-acetate or H-HGEGSFSSELSTILDALAARDFIAWLIATKITDKKKKKK-NH₂ acetate (SEQ ID NO: 7) at a concentration of about 20 mg/mL, histidine buffer at a concentration of about 15 mM, mannitol at a concentration of about 230 mM, and arginine q.s. to provide a pH of about 7.0.

In a further embodiment, the liquid formulations produced according to processes of the comprises an acetate salt of a glucagon-like peptide 2 (GLP-2) analogue having the formula:
(H-HGEGTFSSELATILDALAARDFIAWLIATKITDKKKKKK-NH₂), x(CH₃COOH) or H-HGEGSFSSELSTILDALAARDFIAWLIATKITDKKKKKK-NH₂, x(CH₃COOH), where x is 1.0 to 8.0., at a concentration of about 20 mg/mL, histidine buffer at a concentration of about 15 mM, mannitol at a concentration of about 230 mM and the pH is about 7.0.

In some cases, the liquid formulations produced by the present invention further comprise a preservative. In some cases, the preservative is one selected from the group consisting of benzalkonium chloride, chloro butanol, methyl paraben and potassium sorbate. Generally, the preservative is present in a concentration of about 0.1 % to about 1% of the final formulation volume.

Generally, the formulations of glucagon-like peptide 2 (GLP-2) analogue are administered to patients parenterally, preferably by injection, most typically by subcutaneous injection, intramuscular injection, intravenous injection or intraperitoneal injection. Administration by subcutaneous injection is preferred. The injection may be carried out by a physician, nurse or other healthcare professional, or may be self-administered by the patient. As set out herein, in some aspects, the formulations produced by the processes of the present invention have a viscosity that facilitates loading of the formulation into a pre-filled syringe, an injection pen or other injector device. This may have the advantage of pre-determining the dose of the formulation administered to the patient, e.g. without the need for measurement from a multi-use vial.

### Delivery of the Formulations

In some aspects, the present invention relates to a ready-to-use formulation of GLP-2 analogues, intended for parenteral administration, and suitable for use in e.g. vials, pre-filled syringes, infusion pumps, wearable injectors, disposable auto-injectors or adjustable dose auto-injectors.

### Medical Conditions

The GLP-2 analogue formulations of the present invention are useful as a pharmaceutical agent for preventing or treating an individual suffering from gastrointestinal disorders, including the upper gastrointestinal tract of the oesophagus by administering an effective amount of a GLP-2 analogue, or a salt thereof as described herein. The stomach and intestinal-related disorders include ulcers of any aetiology (e.g., peptic ulcers, drug-induced ulcers, ulcers related to infections or other pathogens), digestion disorders, malabsorption syndromes, short bowel syndrome, cul-de-sac syndrome, inflammatory bowel disease, celiac sprue (for example arising from gluten induced enteropathy or celiac disease), tropical sprue, hypogammaglobulinemic sprue, enteritis, regional enteritis (Crohn's disease), ulcerative colitis, small intestine damage, and chemotherapy induced diarrhoea/mucositis (CID).

### Examples

The following examples are provided to illustrate preferred aspects of the invention and are not intended to limit the scope of the invention. The GLP-2 analogues administered according to the dosage regimes described herein can be made according to the methods such as solid phase peptide synthesis described in WO 2006/117565.

### Analytical methods

For analysis of covalently linked oligomers, a Dionex Ultimate3000 HPLC system running was used with a linear gradient and a flow rate of 0.5 mL/min. The mobile phase as 0.1% TFA in 45% acetonitrile and 55% milli-Q water. A wavelength of 215 nm was used for detection. The injection amount was 4 µg of peptide. The column used was a TSKgel SuperSW2000 (TSK Bioscience) with a 4 µm particle size and dimensions of 300 x 4.6 mm. The overall runtime was 25 mins.

For chemical stability evaluation of the peptide monomer (impurities), a C18 column with an acidic mobile phase and an acetonitrile gradient was used.

Formulations of the ZP1848 drug substance have previously been manufactured using a traditional two-solution formulation process, referred to herein as "Process A". Process A was for glepaglutide led to technical issues associated with excessive foaming, formation of hard lumps of drug substance during dissolution, deviations with visual control, and higher peptide oligomer formation. Foaming and lump formation and the need to use manual stirring can be especially problematic in upscaling. In this Example, we compare the previous process (Process A) with a new process (Process B) with regard to solving the aforementioned technical issues for the traditional previous process.

### Example 1: Process A and B (10 L batch volume)

### Materials and Methods

### Process A:

In a tank, 23.27 g L-histidine, 419 g mannitol and 8.71 g L-arginine were added successively to 5.1 kg Water for Injection (WFI) to obtain an excipient solution (Sol 1, Figure 1). Stirring until a clear and homogenous solution was obtained. 200 g glepaglutide (amount corrected for purity and content) was then added to a separate beaker containing 3.0 kg WFI to obtain an API solution (ZP1848, Figure 1). A further 1.0 L of WFI was used to rinse the peptide transfer bag, and the rinsing water was added to the API solution. Gentle, manual stirring with a spoon until lumps were eliminated and a clear and homogenous API solution was obtained. The resulting API solution was added to the excipient solution to obtain an excipient/API solution (Sol 1 + ZP1848, Figure 1), and pH was adjusted to 7.0 with 1 M acetic acid or 250 mM L-arginine. WFI was added up to 10 L. Gentle stirring until the excipient/API solution was clear and homogenous.

### Process B:

In a tank, 23.27 g L-histidine, 419 g mannitol and 8.71 g L-arginine were added successively to 7.2 kg WFI to obtain an excipient solution (Sol 1, Figure 1). Stirring until a clear and homogenous solution was obtained. 200 g glepaglutide (amount corrected for purity and content) was added directly to the excipient solution to obtain an excipient/API solution (Sol 1 + ZP1848, Figure 1). A further 100 mL WFI was used to rinse the peptide transfer bag. A further 1L WFI was used to rinse the tank sides. The rinsing water was added to the excipient/API solution. Stirring until a clear and homogenous solution was obtained. pH was adjusted to 7.0 with 1 M acetic acid or 250 mM L-arginine. WFI was added up to 10 L. Stirring until a clear and homogenous solution was obtained.

### Results and Discussion

The result of the visual evaluation of the practical aspects of the two processes is shown in Table 1.

**Table 1. Visual in-process controls and evaluation**

| **Batches** | **Dissolution time** | **Foaming issues** | **Failure of visual control for clear solution** | **Lump formation** |
|---|---|---|---|---|
| Process A | < 1 h | Yes | 23% of batches | Major |
| Process B | < 1 h | No | 0% of batches | Minor |

The new process has solved or improved technical issues such as foaming, failure of visual control, and lump formation associated with glepaglutide drug product in the previous manufacturing process A. The process of gentle manual stirring in Process A is also a limitation for upscaling the batch volume from 10 L to larger batch volumes.

The stability results are shown in Tables 2 and 3 for two batches manufactured with Process A and two batches manufactured using Process B.

**Table 2. Oligomer formation during stability studies**

| | **Oligomer content (SEC) at 25°C** | | |
|---|---|---|---|
| | **0 M** | **1M** | **3M** |
| Process A Batch 1 | 0.16 | 0.42 | 0.71 |
| Process A Batch 2 | 0.17 | 0.43 | 0.74 |
| Process B Batch 1 | 0.17 | 0.33 | 0.59 |
| Process B Batch 2 | 0.11 | 0.33 | 0.56 |

The formation of oligomers in glepaglutide drug product is reduced in the new formulation Process B as compared to the previous Process A during stability studies (Table 2).

**Table 3. Impurity formation during stability studies**

| Batches | **Peptide impurities (RP HPLC) at 25°C (%)** | | |
|---|---|---|---|
| | **0 M** | **1M** | **3M** |
| Process A Batch 1 | 1.4 | 3.8 | 8.2 |
| Process A Batch 2 | 1.4 | 3.6 | 7.8 |
| Process B Batch 1 | 1.5 | 3.5 | 7.8 |
| Process B Batch 2 | 1.4 | 3.6 | 7.8 |

The stability of glepaglutide drug product with regard to peptide impurities is identical in the new formulation Process B as compared to the previous Process A (Table 3).

The difference between the two-pot process (Process A) and the one-pot process (Process B) is that Process A requires a gentle, manual stirring with a spoon of the API solution and the excipient/API solution in order to obtain a clear and homogenous excipient/API solution, whereas Process B does not. Due to the proportions between the excipients, WFI and drug substance in Process B, only one pot/tank can be used, which makes this process simpler, easier to handle, more robust for deviations, practicable to upscale, and results in a final drug product with improved stability.

### Example 2: Process B (20 L batch volume)

### Materials and Methods

### Process A:

Process A is described above under Example 1.

### Process B:

In a tank, 46.54 g L-histidine, 0.838 kg mannitol and 17.43 g L-arginine were added successively to 14.4 kg WFI to obtain an excipient solution (Sol 1, Figure 1). Excipient beakers were washed with a maximum of 100 mL WFI each. Stirring until a clear and homogenous solution was obtained. 400 g glepaglutide (amount corrected for purity and content) was added directly to the excipient solution in at least three portions, and preferably six portions, to obtain an excipient/API solution (Sol 1 + ZP1848, Figure 1). A further 300 mL WFI was used to rinse the peptide transfer bags. A further 2L WFI was used to rinse the tank sides. The rinsing water was added to the excipient/API solution. Stirring until a clear and homogenous solution was obtained. pH was adjusted to 7.0 with 1 M acetic acid and/or 250 mM L-arginine. WFI was added up to 20.4 kg. Stirring until a clear and homogenous solution was obtained.

**Table 4. Visual in-process controls and evaluation**

| **Batches** | **Dissolution time** | **Foaming issues** | **Failure of visual control for clear solution** | **Lump formation** |
|---|---|---|---|---|
| Process A | < 1 h | Yes | 23% of batches | Major |
| | | | | |
| Process B (20L) | NA | No | 0% of batches | Minor |

Also in the 20 L batch, the new process B has solved or improved technical issues such as foaming, failure of visual control, and lump formation associated with glepaglutide drug product in the previous manufacturing process A. The process of gentle manual stirring in Process A is also a limitation for upscaling the batch volume from 20 L to larger batch volumes.

The stability results are shown in Tables 5 and 6 for two batches manufactured with

Process A and Process B. In Table 5, the data for the 10 L batch volumes for Process A and Process B is included as a comparison with the upscaling data for the Process B 20 L batch volume.

**Table 5. Oligomer formation during stability studies**

| Process | Batch volume | **Oligomer content (SEC) at 25°C** | | |
|---|---|---|---|---|
| | | **0 M** | **1M** | **3M** |
| Process A Batch 1 | 10 L | 0.16 | 0.42 | 0.71 |
| Process A Batch 2 | 10 L | 0.17 | 0.43 | 0.74 |
| Process B Batch 1 | 10 L | 0.17 | 0.33 | 0.59 |
| Process B Batch 2 | 10 L | 0.11 | 0.33 | 0.56 |
| Process B Batch 3 | 20 L | 0.16 | 0.22 | 0.78 |

A direct comparison between the oligomer content of Process B (10L) and Process B (20L) was not possible, since the total process time (compounding time and filling time combined) at ambient temperature for Process B (20L) is much longer than for Process B (10L), leading to a relative slightly higher oligomer content for the 20L batch (Table 5). Taking the longer process time into considerations, the amount of oligomers is in an acceptable range.

**Table 6. Impurity formation during stability studies**

| Batches | **Peptide impurities (RP HPLC) at 25°C (%)** | | |
|---|---|---|---|
| | **0 M** | **1M** | **3M** |
| Process A Batch 1 | 1.4 | 3.8 | 8.2 |
| Process A Batch 2 | 1.4 | 3.6 | 7.8 |
| Process B Batch 20L | 1.2 | 3.6 | 7.7 |

The stability of glepaglutide drug product with regard to peptide impurities is identical in the new formulation Process B as compared to the previous Process A (Table 6).

### Example 3: Nitrogen as process gas

### Materials and Methods

In each of six separate containers suitable for batch volumes of 15 mL, 34.95 mg L-histidine, 628.5 mg mannitol and 13.07 mg L-arginine were added successively to 10.5 mL of MQ-water to obtain excipient solutions. Stirring until clear and homogeneous solutions were obtained. The solutions were in some of the batches purged for 5 minutes with either nitrogen- or oxygen gas (see Table 7). In accordance with Process B, 300 mg glepaglutide (amount corrected for purity and content) were added to each container. Gentle stirring until lumps were eliminated and clear and homogeneous solutions were obtained. The solutions were in some of the batches overlaid with nitrogen- or oxygen gas (Table 7). pH was adjusted to 7.0 with 1 M acetic acid or 250 mM L-arginine. MnCl₂ and FeCl₃ were added to batch 4-6 (see Table 7) to reach a concentration of 50 + 50 ppm in final batch volume. MQ-water was added up to 15 mL in all formulations. Stirring until a clear and homogenous solution was obtained and in some of the batches under overlay of nitrogen- or oxygen gas (Table 7). Batches filled in vials and in some of the batches with nitrogen- or oxygen gas overlay according to Table 7.

**Table 7. Compositions of formulations in stability study**

| **Batch number** | **pH** | **L-Histidine (mg/mL)** | **Mannitol (mg/mL)** | **L-arginine (mg/mL)** | **Glepaglutide Conc. (mg/mL)** | **Metal ion** | **Metal ion conc (ppm)** | **N₂** | **O₂** |
|---|---|---|---|---|---|---|---|---|---|
| 1 (control) | 7.0 | 2.33 | 41.9 | 0.871 | 20 | None | None | - | - |
| 2 | 7.0 | 2.33 | 41.9 | 0.871 | 20 | None | None | + | - |
| 3 | 7.0 | 2.33 | 41.9 | 0.871 | 20 | None | None | - | + |
| 4 | 7.0 | 2.33 | 41.9 | 0.871 | 20 | MnCl₂ + FeCl₃ | 50+50 | - | - |
| 5 | 7.0 | 2.33 | 41.9 | 0.871 | 20 | MnCl₂ + FeCl₃ | 50+50 | + | - |
| 6 | 7.0 | 2.33 | 41.9 | 0.871 | 20 | MnCl₂ + FeCl₃ | 50+50 | - | + |

### Result and Discussion

The stability results are shown in Tables 8 and 9 for batches with the compositions described in Table 7.

**Table 8. Oligomer formation during stability studies**

| Batches | **Oligomer content (SEC) at 25°C** | | |
|---|---|---|---|
| | **0 M** | **1M** | **3M** |
| 1 | 0.09 | 0.32 | 0.35 |
| 2 | 0.09 | 0.29 | 0.30 |
| 3 | 0.09 | 0.39 | 0.50 |
| 4 | 0.11 | 1.60 | 3.38 |
| 5 | 0.13 | 0.86 | 1.27 |
| 6 | 0.11 | 3.36 | 9.49 |

The formation of oligomers in glepaglutide drug product is reduced in batches with nitrogen gas compared to batches with oxygen- or no gas. The formation of oligomers in glepaglutide drug product is increased in batches with oxygen gas or/and metal salt additive.

**Table 9. Impurity formation during stability studies**

| Batches | **Peptide impurities (RP HPLC) at 25°C (%)** | | |
|---|---|---|---|
| | **0 M** | **1M** | **2M** |
| 1 | 1.6 | 3.1 | 5.0 |
| 2 | 1.3 | 3.0 | 5.0 |
| 3 | 1.6 | 3.1 | 5.0 |
| 4 | 1.6 | 4.1 | 7.1 |
| 5 | 1.7 | 3.6 | 5.5 |
| 6 | 1.6 | 5.9 | 10.4 |

The stability of glepaglutide drug product with regard to peptide impurities is identical in batches with or without nitrogen- or oxygen gas (batch 1-3, Table 9). The stability is decreased in batched with metal salt additives (batch 4-6, Table 9) and is accelerated in combination with oxygen gas but is limited in combination with nitrogen gas.

Glepaglutide drug product is sensitive to oxidation with regard to oligomer formation. The effects of oxidizing agents, such as iron or manganese from e.g. leachable or excipient impurities, can be significantly reduced by eliminating the presence of oxygen by using nitrogen as a process gas. This will make the glepaglutide drug product stability more robust.

## Claims

1. A process for manufacturing a stable liquid pharmaceutical formulation comprising a glucagon-like peptide 2 (GLP-2) analogue, wherein the GLP-2 analogue is represented by the formula:
ZP1848 H-HGEGTFSSELATILDALAARDFIAWLIATKITDKKKKKK-NH₂ (SEQ ID NO: 1) or
ZP1846 H-HGEGSFSSELSTILDALAARDFIAWLIATKITDKKKKKK-NH₂(SEQ ID NO: 7); or a pharmaceutically acceptable salt thereof;
wherein the liquid pharmaceutical formulation comprises the GLP-2 analogue at a concentration of about 2 mg/mL to about 30 mg/mL and excipients comprising (i) a histidine buffer present at a concentration of about 5 mM to about 50 mM, (ii) mannitol as a non-ionic tonicity modifier present at a concentration of about 90 mM to about 360 mM and with (iii) arginine q.s. to provide a formulation having a pH of about 6.6 to about 7.4;
wherein the process comprises:
(a) mixing the excipients with a first volume of water for injection to provide an excipient solution at 70-90% of the volume of a final liquid formulation;
(b) adding the GLP-2 analogue drug substance to the excipient solution to provide a solution of the excipients and the GLP-2 analogue at 70-90% of the volume of a final liquid formulation; and
(c) adjusting the pH and the volume of the liquid composition as needed to provide a liquid formulation at 100% of the volume of the final liquid pharmaceutical formulation;
wherein the process provides a final aqueous liquid pharmaceutical formulation comprising a glucagon-like peptide 2 (GLP-2) analogue.

2. The process of claim 1, wherein the process does not require stirring when the GLP-2 drug substance is added to the excipient solution.

3. The process of claim 1 or claim 2, wherein the GLP-2 analogue drug substance is added as a lyophilized composition.

4. The process of any one of claims 1 to 3, wherein adjusting the volume of the liquid composition in step (c) comprises a first addition of water for injection to adjust the pH and a second addition of water for injection to increase the volume of the liquid composition towards the final volume, optionally wherein adjusting the pH of the liquid composition is carried out by adding arginine and/or acetic acid.

5. The process of any one of the preceding claims, wherein the excipient solution is at about 80% the volume of a final liquid formulation and the first and second additions each add about 10% of the volume of a final liquid formulation.

6. The process of any one of claims 1 to 4, wherein the excipient solution is at about 75% the volume of a final liquid formulation and the first and second additions each add about 15% and about 10% of the volume of a final liquid formulation.

7. The process of any one of the preceding claims, wherein the drug substance is added in 1-5 portions, preferably 3 portions.

8. The process of any one of the preceding claims, further comprising sterile filtering the final liquid pharmaceutical formulation.

9. The process of any one of the preceding claims, further comprising dividing the final liquid pharmaceutical formulation into doses.

10. The process of any one of the preceding claims, further comprising quality control testing the final liquid pharmaceutical formulation and comparing results of the quality control testing with a reference.

11. The process of any one of the preceding claims, wherein the process uses a batch size of 10-50 litres of final liquid pharmaceutical formulation, and optionally a batch size of 10-20 litres of final liquid pharmaceutical formulation, and optionally a batch size of 20-50 litres of final liquid pharmaceutical formulation.

12. The process of any one of the preceding claims, wherein the process is carried out in a 10 litre or a 20 litre tank.

13. The process of any one of the preceding claims, wherein:
(i) mechanical stirring is used in step (b) to dissolve the GLP-2 analogue drug substance, e.g. using a stirrer wing; and/or
(ii) the process is carried out under nitrogen.

14. The process of any one of the preceding claims, wherein the process comprises performing a visual control after step (a) to determine whether the excipients have (fully) dissolved and/or wherein the process comprises performing a visual control after step (b) to determine whether the GLP-2 analogue and the excipients have substantially completely dissolved and/or wherein the process comprises performing a visual control after step (c) to determine whether the process has produced a solution of the GLP-2 analogue and excipients.

15. The process of any one of the preceding claims, wherein step (b) comprises washing a vessel or transfer bag/container that contained the lyophilized GLP-2 analogue to remove any residual GLP-2 analogue.

16. The process of any one of the preceding claims, wherein:
(i) the addition of the GLP-2 analogue in step (b) reduces foaming of the composition comprising the GLP-2 analogue and the excipients and/or formation of residual lumps of the GLP-2 analogue. to secure visual control of clear solution at the end of the process; and/or
(ii) the addition of the GLP-2 analogue in step (b) reduces the level of covalently linked high molecular weight species in the final liquid pharmaceutical formulation.

17. The process of any one of the preceding claims, wherein the GLP-2 analogue is in the form of an acetate salt represented by the formula:
H-HGEGTFSSELATILDALAARDFIAWLIATKITDKKKKKK-NH₂ acetate (SEQ ID NO: 1).

18. The process of any one of claims 1 to 16, wherein the acetate salt of a glucagon-like peptide 2 (GLP-2) analogue has the formula:
(H-HGEGTFSSELATILDALAARDFIAWLIATKITDKKKKKK-NH₂), x(CH₃COOH) where x is 1.0 to 8.0; or
(H-HGEGSFSSELSTILDALAARDFIAWLIATKITDKKKKKK-NH₂), x(CH₃COOH) where x is 1.0 to 8.0.

19. The process of claim 18, wherein in the formula of acetate salt of the glucagon-like peptide 2 analogue, x is from 2.0 to 6.0.

20. The process of any one of the preceding claims, wherein the formulation is stable for at least 18 months when stored at 2-8°C.

21. The process of any one of the preceding claims, wherein the histidine buffer is present at a concentration of about 5 mM to about 25 mM.

22. The process of claim 21, wherein the histidine buffer is present at a concentration of about 15 mM.

23. The process of any one of the preceding claims, wherein mannitol is present as the non-ionic tonicity modifier at a concentration of about 150 mM to about 250 mM.

24. The process of any one of the preceding claims, wherein the formulation has a pH of about 6.8 to about 7.2.

25. The process of claim 24, wherein the formulation has a pH of about 7.0.

26. The process of any one of the preceding claims, wherein the formulation comprises the GLP-2 analogue, or the pharmaceutically acceptable salt thereof; at a concentration of about 20 mg/mL, histidine buffer at a concentration of about 15 mM, mannitol at a concentration of about 230 mM, and arginine q.s. to provide a pH of about 7.0.

27. The process of any one of the preceding claims, wherein the histidine buffer is L-histidine and/or the mannitol is D-mannitol.

28. The process of any one of the preceding claims, further comprising loading the formulation into a pre-filled syringe, an injection pen or an injector device.

29. The process of any one of the preceding claims, wherein the process reduces the formation of covalently bonded oligomeric products of the glucagon-like peptide 2 (GLP-2) analogue in a stable liquid pharmaceutical formulation.

30. Use of a formulation for reducing the formation of covalently bonded oligomeric products of a glucagon-like peptide 2 (GLP-2) analogue, wherein the GLP-2 analogue is represented by the formula:
ZP1848 H-HGEGTFSSELATILDALAARDFIAWLIATKITDKKKKKK-NH₂ (SEQ ID NO: 1) or
ZP1846 H-HGEGSFSSELSTILDALAARDFIAWLIATKITDKKKKKK-NH₂(SEQ ID NO: 7);
or a pharmaceutically acceptable salt thereof;
wherein the liquid pharmaceutical formulation comprises the GLP-2 analogue at a concentration of about 2 mg/mL to about 30 mg/mL and excipients comprising (i) a histidine buffer present at a concentration of about 5 mM to about 50 mM, (ii) mannitol as a non-ionic tonicity modifier present at a concentration of about 90 mM to about 360 mM and with (iii) arginine q.s. to provide a formulation having a pH of about 6.6 to about 7.4;
wherein the use comprises:
(a) mixing the excipients with a first volume of water for injection to provide an excipient solution at 70-90% of the volume of a final liquid formulation;
(b) adding the GLP-2 analogue drug substance to the excipient solution to provide a solution of the excipients and the GLP-2 analogue at 70-90% of the volume of a final liquid formulation; and
(c) adjusting the pH and the volume of the liquid composition as needed to provide a liquid formulation at 100% of the volume of the final liquid pharmaceutical formulation;
wherein the process provides a final aqueous liquid pharmaceutical formulation comprising a glucagon-like peptide 2 (GLP-2) analogue.

## Patentansprüche

1. Verfahren zur Herstellung einer stabilen flüssigen pharmazeutischen Formulierung, umfassend ein Glucagon-ähnliches-Peptid-2- (GLP-2-) Analogon, wobei das GLP-2-Analogon durch folgende Formel dargestellt ist:
ZP1848 H-HGEGTFSSELATILDALAARDFIAWLIATKITDKKKKKK-NH₂ (SEQ ID NO: 1) oder
ZP1846 H-HGEGSFSSELSTILDALAARDFIAWLIATKITDKKKKKK-NH₂ (SEQ ID NO: 7); oder ein pharmazeutisch annehmbares Salz davon;
wobei die flüssige pharmazeutische Formulierung das GLP-2-Analogon in einer Konzentration von etwa 2 mg/ml bis etwa 30 mg/ml und Hilfsstoffe, umfassend (i) einen Histidinpuffer, der in einer Konzentration von etwa 5 mM bis etwa 50 mM vorhanden ist, (ii) Mannit als nichtionischen Tonizitätsmodifikator, der in einer Konzentration von etwa 90 mM bis etwa 360 mM vorhanden ist, und mit (iii) Arginin q. s., umfasst, um eine Formulierung bereitzustellen, die einen pH-Wert von etwa 6,6 bis etwa 7,4 aufweist;
wobei das Verfahren Folgendes umfasst:
(a) Vermischen der Hilfsstoffe mit einem ersten Volumen Wasser zur Injektion, um eine Hilfsmittellösung bei 70 bis 90 % des Volumens einer finalen flüssigen Formulierung bereitzustellen;
(b) Zusetzen der GLP-2-Analogon-Arzneimittelsubstanz zu der Hilfsmittellösung, um eine Lösung der Hilfsmittel und des GLP-2-Analogons bei 70 bis 90 % des Volumens einer finalen flüssigen Zusammensetzung bereitzustellen; und
(c) Einstellen des pH-Werts und des Volumens der flüssigen Zusammensetzung je nach Bedarf, um eine flüssige Formulierung bei 100 % des Volumens der finalen flüssigen pharmazeutischen Formulierung bereitzustellen;
wobei das Verfahren eine finale wässrige flüssige pharmazeutische Formulierung, umfassend ein Glucagon-ähnliches-Peptid-2- (GLP-2-) Analogon, bereitstellt.

2. Verfahren nach Anspruch 1, wobei das Verfahren kein Rühren erfordert, wenn die GLP-2-Arzneimittelsubstanz zu der Hilfsstofflösung zugesetzt wird.

3. Verfahren nach Anspruch 1 oder Anspruch 2, wobei die GLP-2-Analogon-Arzneimittelsubstanz als lyophilisierte Zusammensetzung zugesetzt wird.

4. Verfahren nach einem der Ansprüche 1 bis 3, wobei das Einstellen des Volumens der flüssigen Zusammensetzung in Schritt (c) eine erste Zugabe von Wasser zur Injektion zum Einstellen des pH-Werts und eine zweite Zugabe von Wasser zur Injektion zum Erhöhen des Volumens der flüssigen Zusammensetzung bis auf das finale Volumen umfasst, wobei das Einstellen des pH-Werts der flüssigen Zusammensetzung gegebenenfalls durch Zusetzen von Arginin und/oder Essigsäure durchgeführt wird.

5. Verfahren nach einem der vorangegangenen Ansprüche, wobei die Hilfsstofflösung etwa 80 % des Volumens einer finalen flüssigen Formulierung aufweist und wobei die erste und die zweite Zugabe jeweils etwa 10 % des Volumens einer finalen flüssigen Formulierung hinzufügen.

6. Verfahren nach einem der Ansprüche 1 bis 4, wobei die Hilfsstofflösung etwa 75 % des Volumens einer finalen flüssigen Formulierung aufweist und wobei die erste und die zweite Zugabe jeweils etwa 15 % und etwa 10 % des Volumens einer finalen flüssigen Formulierung hinzufügen.

7. Verfahren nach einem der vorangegangenen Ansprüche, wobei die Arzneimittelsubstanz in 1 bis 5 Portionen, vorzugsweise in 3 Portionen, zugesetzt wird.

8. Verfahren nach einem der vorangegangenen Ansprüche, das weiters das Sterilfiltrieren der finalen flüssigen pharmazeutischen Formulierung umfasst.

9. Verfahren nach einem der vorangegangenen Ansprüche, das weiters das Aufteilen der finalen flüssigen pharmazeutischen Formulierung in Dosen umfasst.

10. Verfahren nach einem der vorangegangenen Ansprüche, das weiters eine Qualitätskontrolle umfasst, in der die finale flüssige pharmazeutische Formulierung getestet und die Ergebnisse der Qualitätskontrolle mit einer Referenz verglichen werden.

11. Verfahren nach einem der vorangegangenen Ansprüche, wobei das Verfahren eine Chargengröße von 10 bis 50 I der finalen flüssigen pharmazeutischen Formulierung und gegebenenfalls eine Chargengröße von 10 bis 20 I der finalen flüssigen pharmazeutischen Formulierung und gegebenenfalls eine Chargengröße von 20 bis 50 I der finalen flüssigen pharmazeutischen Formulierung verwendet.

12. Verfahren nach einem der vorangegangenen Ansprüche, wobei das Verfahren in einem 10-I- oder 20-I-Tank durchgeführt wird.

13. Verfahren nach einem der vorangegangenen Ansprüche, wobei:
(i) mechanisches Rühren in Schritt (b) verwendet wird, um die GLP-2-Analogon-Arzneimittelsubstanz, z. B. unter Verwendung eines Rührflügels, aufzulösen; und/oder
(ii) das Verfahren unter Stickstoff durchgeführt wird.

14. Verfahren nach einem der vorangegangenen Ansprüche, wobei das Verfahren das Durchführen einer visuellen Kontrolle nach Schritt (a) umfasst, um zu bestimmen, ob sich die Hilfsstoffe (vollständig) aufgelöst haben, und/oder wobei das Verfahren das Durchführen einer visuellen Kontrolle nach Schritt (b) umfasst, um zu bestimmen, ob sich das GLP-2-Analogon und die Hilfsstoffe im Wesentlichen vollständig aufgelöst haben, und/oder wobei das Verfahren das Durchführen einer visuellen Kontrolle nach Schritt (c) umfasst, um zu bestimmen, ob das Verfahren eine Lösung des GLP-2-Analogons und der Hilfsstoffe erzeugt hat.

15. Verfahren nach einem der vorangegangenen Ansprüche, wobei Schritt (b) das Waschen eines Gefäßes oder eines Überführungsbeutels/-behälters umfasst, das/der das lyophilisierte GLP-2-Analogon enthalten hat, um jegliche Rückstände des GLP-2-Analogons zu entfernen.

16. Verfahren nach einem der vorangegangenen Ansprüche, wobei:
(i) die Zugabe des GLP-2-Analogons in Schritt (b) das Schäumen der Zusammensetzung, umfassend das GLP-2-Analogon und die Hilfsstoffe, und/oder die Bildung von Rückstandsklümpchen des GLP-2-Analogons reduziert, um eine visuelle Kontrolle der klaren Lösung am Ende des Verfahrens zu gewährleisten; und/oder
(ii) die Zugabe des GLP-2-Analogons in Schritt (b) die Menge an kovalent gebundener Spezies mit hohem Molekulargewicht in der finalen flüssigen pharmazeutischen Formulierung reduziert.

17. Verfahren nach einem der vorangegangenen Ansprüche, wobei das GLP-2-Analogon in Form eines Acetatsalzes vorliegt, das durch folgende Formel dargestellt ist:
H-HGEGTFSSELATILDALAARDFIAWLIIATKITDKKKKKK-NH₂-Acetat (SEQ ID NO: 1).

18. Verfahren nach einem der Ansprüche 1 bis 16, wobei das Acetatsalz eines Glucagonähnliches-Peptid-2- (GLP-2-) Analogons folgende Formel aufweist:
(H-HGEGTFSSELATILDALAARDFIAWLIATKITDKKKKKK-NH₂), x(CH₃COOH), worin x = 1,0 bis 8,0 ist; oder
(H-HGEGSFSSELSTILDALAARDFIAWLIATKITDKKKKKK-NH₂), x(CH₃COOH), worin x = 1,0 bis 8,0 ist.

19. Verfahren nach Anspruch 18, wobei in der Formel des Acetatsalzes des Glucagonähnliches-Peptid-2-Analogons x = 2,0 bis 6,0 ist.

20. Verfahren nach einem der vorangegangenen Ansprüche, wobei die Formulierung für zumindest 18 Monate stabil ist, wenn sie bei 2 bis 8 °C gelagert wird.

21. Verfahren nach einem der vorangegangenen Ansprüche, wobei der Histidinpuffer in einer Konzentration von etwa 5 mM bis etwa 25 mM vorhanden ist.

22. Verfahren nach Anspruch 21, wobei der Histidinpuffer in einer Konzentration von etwa 15 mM vorhanden ist.

23. Verfahren nach einem der vorangegangenen Ansprüche, wobei Mannit als nichtionischer Tonizitätsmodifikator in einer Konzentration von etwa 150 mM bis etwa 250 mM vorhanden ist.

24. Verfahren nach einem der vorangegangenen Ansprüche, wobei die Formulierung einen pH-Wert von etwa 6,8 bis etwa 7,2 aufweist.

25. Verfahren nach Anspruch 24, wobei die Formulierung einen pH-Wert von etwa 7,0 aufweist.

26. Verfahren nach einem der vorangegangenen Ansprüche, wobei die Formulierung das GLP-2-Analogon oder das pharmazeutisch annehmbare Salz davon in einer Konzentration von etwa 20 mg/ml, einen Histidinpuffer in einer Konzentration von etwa 15 mM, Mannit in einer Konzentration von etwa 230 mM und Arginin q. s. umfasst, um einen pH-Wert von etwa 7,0 bereitzustellen.

27. Verfahren nach einem der vorangegangenen Ansprüche, wobei der Histidinpuffer L-Histidin ist und/oder der Mannit D-Mannit ist.

28. Verfahren nach einem der vorangegangenen Ansprüche, das weiters das Laden der Formulierung in eine vorgefüllte Spritze, einen Injektionsstift oder eine Injektionsvorrichtung umfasst.

29. Verfahren nach einem der vorangegangenen Ansprüche, wobei das Verfahren die Bildung von kovalent gebundenen Oligomerprodukten des Glucagon-ähnliches-Peptid-2-(GLP-2-) Analogons in einer stabilen flüssigen pharmazeutischen Formulierung reduziert.

30. Verwendung einer Formulierung zur Reduktion der Bildung von kovalent gebundenen Oligomerprodukten eines Glucagon-ähnliches-Peptid-2- (GLP-2-) Analogons, wobei das GLP-2-Analogon durch folgende Formel dargestellt ist:
ZP1848 H-HGEGTFSSELATILDALAARDFIAWLIATKITDKKKKKK-NH₂ (SEQ ID NO: 1) oder
ZP11846 H-HGEGSFSSELSTILDALAARDFIAWLIATKITDKKKKKK-NH_{2 (}SEQ ID NO: 7); oder eines pharmazeutisch annehmbaren Salzes davon;
wobei die flüssige pharmazeutische Formulierung das GLP-2-Analogon in einer Konzentration von etwa 2 mg/ml bis etwa 30 mg/ml und Hilfsstoffe, umfassend (i) einen Histidinpuffer, der in einer Konzentration von etwa 5 mM bis etwa 50 mM vorhanden ist, (ii) Mannit als nichtionischen Tonizitätsmodifikator, der in einer Konzentration von etwa 90 mM bis etwa 360 mM vorhanden ist, und mit (iii) Arginin q. s., umfasst, um eine Formulierung bereitzustellen, die einen pH-Wert von etwa 6,6 bis etwa 7,4 aufweist;
wobei die Verwendung Folgendes umfasst:
(a) Vermischen der Hilfsstoffe mit einem ersten Volumen Wasser zur Injektion, um eine Hilfsmittellösung bei 70 bis 90 % des Volumens einer finalen flüssigen Formulierung bereitzustellen;
(b) Zusetzen der GLP-2-Analogon-Arzneimittelsubstanz zu der Hilfsmittellösung, um eine Lösung der Hilfsmittel und des GLP-2-Analogons bei 70 bis 90 % des Volumens einer finalen flüssigen Zusammensetzung bereitzustellen; und
(c) Einstellen des pH-Werts und des Volumens der flüssigen Zusammensetzung je nach Bedarf, um eine flüssige Formulierung bei 100 % des Volumens der finalen flüssigen pharmazeutischen Formulierung bereitzustellen;
wobei das Verfahren eine finale wässrige flüssige pharmazeutische Formulierung, umfassend ein Glucagon-ähnliches-Peptid-2- (GLP-2-) Analogon, bereitstellt.

## Revendications

1. Procédé de fabrication d'une formulation pharmaceutique liquide stable comprenant un analogue du GLP-2 (glucagon-like peptide 2), dans lequel l'analogue du GLP-2 est représenté par la formule :
ZP1848 H-HGEGTFSSELATILDALAARDFIAWLIATKITDKKKKKK-NH₂ (SEQ ID NO: 1) ou
ZP1846 H-HGEGSFSSELSTILDALAARDFIAWLIATKITDKKKKKK-NH₂ (SEQ ID NO: 7) ;
ou un sel de celui-ci pharmaceutiquement acceptable ;
dans lequel la formulation pharmaceutique liquide comprend l'analogue du GLP-2 à une concentration d'environ 2 mg/ml à environ 30 mg/ml et des excipients comprenant (i) un tampon histidine présent à une concentration d'environ 5 mM à environ 50 mM, (ii) du mannitol en tant que modificateur de tonicité non ionique présent à une concentration d'environ 90 mM à environ 360 mM et avec (iii) de l'arginine q.s. pour obtenir une formulation possédant un pH d'environ 6,6 à environ 7,4 ;
dans lequel le procédé comprend :
(a) le mélange des excipients avec un premier volume d'eau pour injection afin d'obtenir une solution d'excipient à 70-90 % du volume d'une formulation liquide finale ;
(b) l'ajout de la substance médicamenteuse d'analogue du GLP-2 à la solution d'excipient afin d'obtenir une solution des excipients et de l'analogue du GLP-2 à 70-90 % du volume d'une formulation liquide finale ; et
(c) l'ajustement du pH et du volume de la composition liquide tel que nécessaire afin d'obtenir une formulation liquide à 100 % du volume de la formulation pharmaceutique liquide finale ;
dans lequel le procédé permet d'obtenir une formulation pharmaceutique liquide aqueuse finale comprenant un analogue du GLP-2 (glucagon-like peptide 2).

2. Procédé selon la revendication 1, dans lequel le procédé ne nécessite pas d'agitation lorsque la substance médicamenteuse de GLP-2 est ajoutée à la solution d'excipient.

3. Procédé selon la revendication 1 ou la revendication 2, dans lequel la substance médicamenteuse d'analogue du GLP-2 est ajoutée sous forme de composition lyophilisée.

4. Procédé selon l'une quelconque des revendications 1 à 3, dans lequel l'ajustement du volume de la composition liquide à l'étape (c) comprend un premier ajout d'eau pour injection pour ajuster le pH et un deuxième ajout d'eau pour injection pour augmenter le volume de la composition liquide jusqu'au volume final, facultativement dans lequel l'ajustement du pH de la composition liquide est réalisé en ajoutant de l'arginine ou de l'acide acétique.

5. Procédé selon l'une quelconque des revendications précédentes, dans lequel la solution d'excipient est à environ 80 % du volume d'une formulation liquide finale et les premier et deuxième ajouts ajoutent chacun environ 10 % du volume d'une formulation liquide finale.

6. Procédé selon l'une quelconque des revendications 1 à 4, dans lequel la solution d'excipient est à environ 75 % du volume d'une formulation liquide finale et les premier et deuxième ajouts ajoutent chacun environ 15 % et environ 10 % du volume d'une formulation liquide finale.

7. Procédé selon l'une quelconque des revendications précédentes, dans lequel la substance médicamenteuse est ajoutée en 1 à 5 parties, de préférence 3 parties.

8. Procédé selon l'une quelconque des revendications précédentes, comprenant en outre la filtration stérile de la formulation pharmaceutique liquide finale.

9. Procédé selon l'une quelconque des revendications précédentes, comprenant en outre la division de la formulation pharmaceutique liquide finale en doses.

10. Procédé selon l'une quelconque des revendications précédentes, comprenant en outre un test de contrôle qualité de la formulation pharmaceutique liquide finale et la comparaison des résultats du test de contrôle qualité avec une référence.

11. Procédé selon l'une quelconque des revendications précédentes, dans lequel le procédé utilise une taille de lot de 10 à 50 litres de formulation pharmaceutique liquide finale, et facultativement une taille de lot de 10 à 20 litres de formulation pharmaceutique liquide finale, et facultativement une taille de lot de 20 à 50 litres de formulation pharmaceutique liquide finale.

12. Procédé selon l'une quelconque des revendications précédentes, dans lequel le procédé est réalisé dans une cuve de 10 litres ou de 20 litres.

13. Procédé selon l'une quelconque des revendications précédentes, dans lequel :
(i) une agitation mécanique est utilisée à l'étape (b) pour dissoudre la substance médicamenteuse d'analogue du GLP-2, par exemple en utilisant un agitateur à ailettes ; et/ou
(ii) le procédé est réalisé sous azote.

14. Procédé selon l'une quelconque des revendications précédentes, dans lequel le procédé comprend la réalisation d'un contrôle visuel après l'étape (a) pour déterminer si les excipients se sont (complètement) dissous et/ou dans lequel le procédé comprend la réalisation d'un contrôle visuel après l'étape (b) pour déterminer si l'analogue du GLP-2 et les excipients se sont essentiellement complètement dissous et/ou dans lequel le procédé comprend la réalisation d'un contrôle visuel après l'étape (c) pour déterminer si le procédé a produit une solution de l'analogue du GLP-2 et des excipients.

15. Procédé selon l'une quelconque des revendications précédentes, dans lequel l'étape (b) comprend le lavage d'un récipient ou d'une poche/d'un conteneur de transfert qui a contenu l'analogue du GLP-2 lyophilisé afin d'éliminer tout analogue du GLP-2 résiduel.

16. Procédé selon l'une quelconque des revendications précédentes, dans lequel :
(i) l'ajout de l'analogue du GLP-2 à l'étape (b) réduit la formation de mousse dans la composition comprenant l'analogue du GLP-2 et les excipients et/ou la formation de grumeaux résiduels de l'analogue du GLP-2 pour garantir le contrôle visuel de la solution transparente à la fin du procédé ; et/ou
(ii) l'ajout de l'analogue du GLP-2 à l'étape (b) réduit le taux d'espèces de haut poids moléculaire à liaisons covalentes dans la formulation pharmaceutique liquide finale.

17. Procédé selon l'une quelconque des revendications précédentes, dans lequel l'analogue du GLP-2 est sous la forme d'un sel d'acétate représenté par la formule :
H-HGEGTFSSELATILDALAARDFIAWLIATKITDKKKKKK-NH₂ acétate (SEQ ID NO: 1).

18. Procédé selon l'une quelconque des revendications 1 à 16, dans lequel le sel d'acétate d'un analogue du GLP-2 (glucagon-like peptide 2) possède la formule :
(H-HGEGTFSSELATILDALAARDFIAWLIATKITDKKKKKK-NH₂), x(CH₃COOH) où x est 1,0 à 8,0 ; ou
(H-HGEGSFSSELSTILDALAARDFIAWLIATKITDKKKKKK-NH₂), x(CH₃COOH) où x est 1,0 à 8,0.

19. Procédé selon la revendication 18 dans lequel, dans la formule du sel d'acétate de l'analogue du glucagon-like peptide 2, x est compris entre 2,0 et 6,0.

20. Procédé selon l'une quelconque des revendications précédentes, dans lequel la formulation est stable pendant au moins 18 mois lorsqu'elle est conservée entre 2 et 8 °C.

21. Procédé selon l'une quelconque des revendications précédentes, dans lequel le tampon histidine est présent à une concentration d'environ 5 mM à environ 25 mM.

22. Procédé selon la revendication 21, dans lequel le tampon histidine est présent à une concentration d'environ 15 mM.

23. Procédé selon l'une quelconque des revendications précédentes, dans lequel du mannitol est présent en tant que modificateur de tonicité non ionique à une concentration d'environ 150 mM à environ 250 mM.

24. Procédé selon l'une quelconque des revendications précédentes, dans lequel la formulation possède un pH d'environ 6,8 à environ 7,2.

25. Procédé selon la revendication 24, dans lequel la formulation possède un pH d'environ 7,0.

26. Procédé selon l'une quelconque des revendications précédentes, dans lequel la formulation comprend l'analogue du GLP-2, ou le sel de celui-ci pharmaceutiquement acceptable ; à une concentration d'environ 20 mg/ml, le tampon histidine à une concentration d'environ 15 mM, du mannitol à une concentration d'environ 230 mM, et de l'arginine q.s. pour obtenir un pH d'environ 7,0.

27. Procédé selon l'une quelconque des revendications précédentes, dans lequel le tampon histidine est la L-histidine et/ou le mannitol est le D-mannitol.

28. Procédé selon l'une quelconque des revendications précédentes, comprenant en outre le chargement de la formulation dans une seringue préremplie, un stylo injecteur ou un dispositif d'injection.

29. Procédé selon l'une quelconque des revendications précédentes, dans lequel le procédé réduit la formation de produits oligomériques à liaisons covalentes de l'analogue du GLP-2 (glucagon-like peptide 2) dans une formulation pharmaceutique liquide stable.

30. Utilisation d'une formulation pour réduire la formation de produits oligomériques à liaisons covalentes d'un analogue du GLP-2 (glucagon-like peptide 2), dans laquelle l'analogue du GLP-2 est représenté par la formule :
ZP1848 H-HGEGTFSSELATILDALAARDFIAWLIATKITDKKKKKK-NH₂ (SEQ ID NO: 1) ou
ZP1846 H-HGEGSFSSELSTILDALAARDFIAWLIATKITDKKKKKK-NH₂ (SEQ ID NO: 7) ;
ou un sel de celui-ci pharmaceutiquement acceptable ;
dans lequel la formulation pharmaceutique liquide comprend l'analogue du GLP-2 à une concentration d'environ 2 mg/ml à environ 30 mg/ml et des excipients comprenant (i) un tampon histidine présent à une concentration d'environ 5 mM à environ 50 mM, (ii) du mannitol en tant que modificateur de tonicité non ionique présent à une concentration d'environ 90 mM à environ 360 mM et avec (iii) de l'arginine q.s. pour obtenir une formulation possédant un pH d'environ 6,6 à environ 7,4 ;
dans laquelle l'utilisation comprend :
(a) le mélange des excipients avec un premier volume d'eau pour injection afin d'obtenir une solution d'excipient à 70-90 % du volume d'une formulation liquide finale ;
(b) l'ajout de la substance médicamenteuse d'analogue du GLP-2 à la solution d'excipient afin d'obtenir une solution des excipients et de l'analogue du GLP-2 à 70-90 % du volume d'une formulation liquide finale ; et
(c) l'ajustement du pH et du volume de la composition liquide tel que nécessaire afin d'obtenir une formulation liquide à 100 % du volume de la formulation pharmaceutique liquide finale ;
dans lequel le procédé permet d'obtenir une formulation pharmaceutique liquide aqueuse finale comprenant un analogue du GLP-2 (glucagon-like peptide 2).
